# EUROPEAN PATENT SPECIFICATION

(11) **EP 2 308 459 B1**
(45) Date of publication and mention of the grant of the patent: **09.08.2017**
(21) Application number: 09754852.3
(22) Date of filing: 01.06.2009
(51) Int. Cl.: A61K 8/81, A61K 8/06, A61K 8/86, A61Q 1/02, A61Q 1/10, A61Q 5/06, A61Q 19/00, A61K 8/73

(54) **OIL IN WATER TYPE TOPICAL AGENT FOR SKIN**
TOPISCHES HAUTPRÄPARAT VOM ÖL-IN-WASSER-TYP
AGENT TOPIQUE POUR LA PEAU DU TYPE HUILE DANS L'EAU

(30) Priority: 30.05.2008 JP 2008143428
(43) Date of publication of application: 13.04.2011
(73) Proprietor: Shiseido Company Ltd., Chuo-ku Tokyo 104-0061 (JP)
(72) Inventor: SUZUKI Kazunobu, Yokohama-shi Kanagawa 224-8558 (JP); SUGAHARA Misato, Yokohama-shi Kanagawa 224-8558 (JP); ISHINO Hirokazu, Yokohama-shi Kanagawa 224-8558 (JP); MASUDA Masahiko, Yokohama-shi Kanagawa 224-8558 (JP)
(74) Representative: Lippert Stachow Patentanwälte Rechtsanwälte
(86) International application number: PCT/JP2009/059958
(87) International publication number: WO 2009/145344

(56) References cited:
- EP-A2- 1 514 537
- WO-A2-2004/075621
- JP-A- 2005 325 088
- JP-A- 2007 051 107
- JP-A- 2007 051 107
- JP-A- 2007 186 471
- JP-A- 2007 262 229
- JP-A- 2007 302 583

## Description

### RELATED APPLICATIONS

This application claims the priority of Japanese Patent Application No. 2008-143428 filed on May 30, 2008.

### FIELD OF THE INVENTION

This invention relates to an oil-in-water external skin preparation having thread-forming property, providing richness, a fresh feeling, and no stickiness when the applied preparation gets dry, penetrating well into skin, and being excellent in time-dependent stability of the thread-forming property.

### BACKGROUND OF THE INVENTION

In an external skin preparation, various water-soluble polymers are incorporated for improving stability and achieving moisturizing effect. Some of these water-soluble polymers have thread-forming property though there is variability in the degree of the property. However, a preparation having the thread-forming property provided stickiness. Thus, in a conventional external skin preparation which incorporated a water-soluble polymer having thread-forming property, there has been an attempt to suppress the thread-forming property as much as possible.

A polyacrylic acid or a metal salt thereof, which is a water-soluble polymer, can provide a fresh feeling in use and an elasticity to the skin when it is incorporated in a preparation. However, it has been known that even a small amount of the polyacrylic acid or metal salt thereof gives thread-forming property. Moreover, it has been known that this compound penetrates into skin poorly, provides stickiness when the applied preparation gets dry, and has a poor time-dependent stability of the thread-forming property.

On the other hand, a polyacrylic acid or a metal salt thereof has been known to be incorporated in dentifrices (see, Patent Literature 1), adhesive skin patches, denture fixatives, and so on, by taking advantage of its marked thread-forming property and adherence property.

Also, as an external preparation incorporating sodium polyacrylate, a hair dressing preparation using a base with thread-forming property is disclosed (see, Patent Literature 2). As the hair dressing preparation includes waxes as essential components and is emulsified with surfactants, it would provide stickiness if it was applied to an external skin preparation.

Also, as an external skin preparation incorporating sodium polyacrylate, an oil-in-water emulsion is disclosed (see, Patent Literature 3). As this oil-in-water emulsion includes virtually no surfactant as an emulsifier, it is characterized by the usability of non-stickiness and light spreadability. However, in this oil-in-water emulsion prepared with sodium polyacrylate and the specified emulsifying aids, it was difficult to achieve sufficient thread-forming property and fresh feeling, and there have been problems in the time-dependent stability of thread-forming property even when the thread-forming property and richness could be achieved.

EP 1514537A2 discloses an O/W cosmetic composition comprising 2 % PEG6000, 0.05% alkyl-modified carboxyvinyl polymer and 0.5% ammonium polyacrylate.

JP 2007 051107A discloses hair cosmetics comprising a polyacrylic acid metal salt, hydrophilic copolymers, polyethyleneglycol and dimethyl siloxane.

JP 2007 262229A discloses a topical preparation in the form of a water based pigment dispersion comprising an inorganic pigment, nonionic surfactant and sodium polyacrylate which shows improved time-dependent dispersion stability.

JP 2007 302583A discloses a water-in-oil type comprising copolymers.

Patent Literature 1: Japanese unexamined patent publication No. 2000-319150
Patent Literature 2: Japanese unexamined patent publication No. H10-45546
Patent Literature 3: Japanese unexamined patent publication No. H6-165932

### DISCLOSURE OF THE INVENTION

### PROBLEM TO BE SOLVED BY THE INVENTION

A preparation having a adequate thread-forming property can be expected to provide distinctive richness and luxurious feeling during being spread on skin. However, as a conventional preparation having such property resulted in causing stickiness when the applied preparation got dry, there has never been an attempt to provide an external skin preparation with the thread-forming property. Also, in such external skin preparation incorporating a water-soluble polymer having thread-forming property, it could not be avoided that the usability was deteriorated as stickiness caused and so on and that the time-dependent stability of thread-forming property became poorer.

The present invention was made in view of the above-mentioned conventional art, and the object is to provide an oil-in-water external skin preparation having the thread-forming property and time-dependent stability of the thread-forming property, penetrating well into skin, and providing an excellent richness, a fresh feeling, and no stickiness even when the applied preparation gets dry.

### MEANS TO SOLVE THE PROBLEM

The present inventors have studied diligently to solve the problems. As a result, they found that an external skin preparation containing a specific amount of sodium polyacrylate with thread-forming property and richness can achieve an excellent fresh feeling and emulsion stability by incorporating a specific amount of alkyl-modified carboxyvinyl polymer, the external skin preparation can have time-dependent stability of the thread-forming property by further incorporating a specific amount of polyethylene glycol in a specific range of molecular weight, and, by further incorporating a specific amount of oil, the external skin preparation can be an oil-in-water external skin preparation which provides the usability of non-stickiness even when the applied preparation gets dry, thus leading to completion of the present invention.

Thus, the oil-in-water external skin preparation according to the present invention comprises the following components (a) to (d):
(a) 0.01 to 3.0 % by mass of sodium polyacrylate;
(b) 0.01 to 1.0 % by mass of alkyl-modified carboxyvinyl polymer;
(c) 1.0 to 10.0 % by mass of polyethylene glycol having an average molecular weight of 1000 or more to 50000 or less; and
(d) 0.5 to 20.0 % by mass of oil; and
wherein the oil-in-water external skin preparation has thread-forming property.

It is preferred that the oil-in-water external skin preparation of the present invention further comprises a component (e): a water-soluble polymer other than the components (a) and (b), especially other than polyacrylic acid or a metal salt thereof.

In the above-mentioned oil-in-water external skin preparation of the present invention, it is also preferred that the length of formed thread at a velocity of 30 cm/min. is 20 mm or more to 200 mm or less, measured as claimed.

In the above-mentioned oil-in-water external skin preparation of the present invention, it is also preferred that the component (e) is one or more selected from succinoglucan, polyacrylamide, carboxyvinyl polymer, and xanthan gum.

In the above-mentioned oil-in-water external skin preparation of the present invention, it is preferred that the oil-in-water external skin preparation further comprises a component (f): one or more of hydrophilic nonionic surfactants or hydrophilic anionic surfactants.

### EFFECT OF THE INVENTION

The oil-in-water external skin preparation penetrating well into skin, having thread-forming property and a good usability without stickiness even when the applied preparation gets dry, and being excellent in time-dependent stability of thread-forming property can be obtained by incorporating (a) 0.01 to 3.0 % by mass of sodium polyacrylate, (b) 0.01 to 1.0 % by mass of alkyl-modified carboxyvinyl polymer, (c) 1.0 to 10.0 % by mass of polyethylene glycol having an average molecular weight of 1000 or more to 50000 or less, and (d) 0.5 to 20.0 % by mass of oil.

### BEST MODE FOR CARRYING OUT THE INVENTION

Hereinafter, the preferable embodiments of the present invention will be illustrated.

### ((a) Sodium polyacrylate)

In the present invention, by incorporating sodium polyacrylate in the oil-in-water external skin preparation, the preparation is given thread-forming property which can be sensed when it is taken in hands and applied to skin. Owing to the incorporation of sodium polyacrylate, the oil-in-water external skin preparation according to the present invention has thread-forming property and also can provide a distinctive richness and luxurious feeling during being spread on skin.

Examples of sodium polyacrylate (or respective polyacrylic acid) include AQUALIC L-YS and AQUALIC L-HL which are manufactured by Nippon Shokubai Co., Ltd.; and JULIMAR AC-10NP, JULIMAR AC-10P, ARONBIS S, and ARONBIS SS which are manufactured by Nihon Junyaku Co., Ltd.

The amount of sodium polyacrylate is 0.01 to 3.0 % by mass, preferably 0.03 to 2.0 % by mass, and more preferably 0.03 to 1.0 % by mass. When the amount of sodium polyacrylate is too small, sufficient thread-forming property and richness cannot be provided. When the amount is too large, the preparation penetrates into skin poorly during application, and stickiness is also caused.

As a result that the present inventors studied various polyacrylic acids or metal salts thereof, they found that, despite the kind or amount of polyacrylic acid or metal salt thereof, the oil-in-water external skin preparation having an excellent usability can be produced when the length of formed thread is in a specific range.

Specifically, in the oil-in-water external skin preparation according to the present invention, the length of formed thread at a velocity of 30 cm/min. is preferably 20 mm or more to 200 mm or less, and in particular 20 mm or more to 100 mm or less, wherein the length of formed thread is measured as mentioned below. When the length of formed thread is in this range, particularly distinctive richness and luxurious feeling can be provided during spreading the preparation on skin. When the length of formed thread is too short, the thread-forming property may not be sensed, and a sufficient richness may not be provided.

In this context, the length of formed thread at a velocity of 30 cm/min. means the length measured as follows: the surface of a sample is flatted and adjusted to be 30 °C; the surface of the sample is lightly contacted with a round disk having a diameter of about 1 cm uniformly; the sample is dropped vertically at a velocity of 30 cm/min.; and the state of forming threads is observed to measure the length of thread when the thread breaks.

### ((b) Alkyl-modified carboxyvinyl polymer)

For alkyl-modified carboxyvinyl polymer used in the present invention, an acrylic acid/alkyl methacrylate copolymer having a molecular weight of 500000 to 3000000 represented by the following formula (1) is particularly preferred; however, it is not limited in particular.

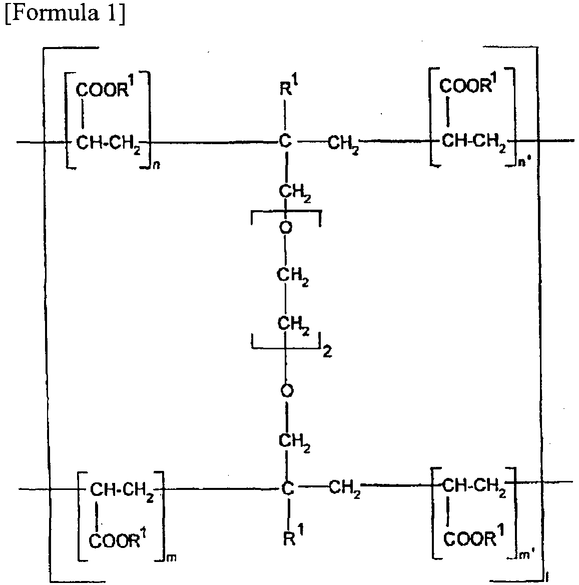

In the formula (1), R¹s are alkyl groups having 10 to 30 carbon atoms and a part of R¹s may be hydrogen atoms; n, n', m, and m' respectively represent any integers so that n + n' + m + m' is 40 to 100; and 1 represents any integer so that the molecular weight is in a specified range.

Specific examples of alkyl-modified carboxyvinyl polymer include Pemulen TR-1, Pemulen TR-2, and Carbopol 1342 which are manufactured by Lubrizol Advanced Materials, Inc.

The amount of alkyl-modified carboxyvinyl polymer is 0.01 to 1.0 % by mass, preferably 0.03 to 0.5 % by mass, and more preferably 0.03 to 0.3 % by mass. When the amount of alkyl-modified carboxyvinyl polymer is too small, the fresh feeling and the emulsion stability are not sufficient. When it is too large, the preparation penetrates into skin poorly, and stickiness is caused.

The oil-in-water external skin preparation of the present invention can achieve a fresh feeling and a good emulsion stability by being emulsified virtually with the alkyl-modified carboxyvinyl polymer. However, emulsification with surfactants may be used in combination with the emulsification with alkyl-modified carboxyvinyl polymer as long as the effects of the present invention are not deteriorated.

### ((c) Polyethylene glycol having an average molecular weight of 1000 or more to 50000 or less)

Polyethylene glycol used in the present invention has an average molecular weight of 1000 or more to 50000 or less, and preferably 1000 or more to 20000 or less. When the average molecular weight of polyethylene glycol is too small, the time-dependent stability of thread-forming property cannot be improved. When it is too large, the preparation penetrates into skin poorly, and stickiness is caused.

Examples of polyethylene glycol having an average molecular weight of 1000 or more to 50000 or less include Polyethylene glycol 1000, Polyethylene glycol 1500, Polyethylene glycol 6000, Polyethylene glycol 11000, and Polyethylene glycol 20000 which are manufactured by TOHO Chemical Industry Co., LTD. or NOF CORPORATION.

The amount of polyethylene glycol having an average molecular weight of 1000 or more to 50000 or less is 1.0 to 10.0 % by mass, and preferably 1.0 to 5.0 % by mass. When the amount of polyethylene glycol having an average molecular weight of 1000 or more to 50000 or less is too small, the time-dependent stability of thread-forming property cannot be improved. When it is too large, the preparation penetrates into skin poorly, and stickiness is caused.

### ((d) Oil)

An oil incorporated in the oil phase used in the present invention is an oil agent usually used in cosmetic emulsions. Any of a natural animal oil, a natural vegetable oil, and a synthetic oil can be used as such oil agent, and specific examples include liquid, paste, and solid hydrocarbon oils such as liquid paraffin and squalane; waxes; higher fatty acids; higher alcohols; esters; glycerides; and silicone oils.

Examples of liquid oils include linseed oil, camellia oil, macadamia nut oil, corn oil, mink oil, olive oil, avocado oil, sasanqua oil, castor oil, safflower oil, apricot kernel oil, cinnamon oil, jojoba oil, grape oil, sunflower oil, meadowfoam oil, almond oil, rapeseed oil, sesame oil, wheat germ oil, rice germ oil, rice bran oil, cottonseed oil, soybean oil, peanut oil, tea seed oil, evening primrose oil, egg oil, beef bone tallow, liver oil, triglycerin, glyceryl trioctanoate, pentaerythritol tetraoctanoate, and glyceryl triisopalmitate.

Examples of solid oils include cacao butter, coconut oil, palm oil, palm kernel oil, beef tallow, mutton tallow, pork tallow, horse fat, hydrogenated oil, hydrogenated castor oil, Japan wax, and shea butter. Examples of waxes include beeswax, candelilla wax, cotton wax, carnauba wax, bayberry wax, insect wax, spermaceti, montan wax, bran wax, lanolin, reduced lanolin, hard lanolin, kapok wax, sugarcane wax, jojoba wax, and shellac wax.

Examples of ester oils include octanoate esters such as cetyl octanoate; laurate esters such as hexyl laurate; myristate esters such as isopropyl myristate and octyldodecyl myristate; palmitate esters such as octyl palmitate; stearate esters such as isocetyl stearate; isostearate esters such as isopropyl isostearate; isopalmitate esters such as octyl isopalmitate; oleate esters such as isodecyl oleate; adipate diesters such as diisopropyl adipate; sebacate diesters such as diethyl sebacate; and malate diesters such as diisostearyl malate.

Examples of hydrocarbon oils include liquid paraffin, hydrogenated polydecene, ozokerite, squalane, squalene, pristane, paraffin, isoparaffin, ceresin, petrolatum, and microcrystalline wax. Examples of silicone oils include chainlike silicones such as dimethylpolysiloxane, methylphenylpolysiloxane, and methylhydrogenpolysiloxane; and cyclic silicones such as octamethylcyclotetrasiloxane, decamethylcyclopentasiloxane, and dodecamethylcyclohexasiloxane.

It is preferred that the oil of the component (d) is one or more selected from squalane, liquid paraffin, hydrogenated polydecene, dimethicone, glyceryl triethylhexanoate, dimethylpolysiloxane, methylphenylpolysiloxane, and decamethylcyclopentasiloxane.

The amount of these oils is 0.5 to 20.0 % by mass in the emulsion, and preferably 1.0 to 10.0 % by mass in terms of the usability. When the amount of oils is too small, the fresh feeling is deteriorated, and stickiness is caused when the applied preparation gets dry. When it is too large, there are problems in the stability, and an oily feeling is caused.

In the oil-in-water external skin preparation of the present invention, as necessary, the following components usually used in external skin preparations can be incorporated arbitrarily in addition to the above-mentioned essential components (a) to (d).

### ((e) Water-soluble viscosity-increasing polymer other than the components (a) and (b))

In the oil-in-water external skin preparation of the present invention, it is preferred that a water-soluble viscosity-increasing polymer other than the components (a) and (b), especially other than polyacrylic acid or a metal salt thereof, is contained as the component (e). By incorporating the component (e), the usability such as richness and the time-dependent stability of thread-forming property can be further improved.

Examples of water-soluble polymer include plant-derived polymers such as gum arabic, tragacanth, galactan, carob gum, guar gum, karaya gum, carrageenan, xanthan gum, pectin, agar, quince seed (quince), starch (rice, corn, potato, and wheat), and algae colloid (brown algae extract); microorganism-derived polymers such as dextran, succinoglucan, and pullulan; animal-derived polymers such as collagen, casein, albumin, and gelatin; and starch-based polymers such as carboxymethyl starch and methylhydroxypropyl starch.

It also includes cellulose polymers such as methyl cellulose, nitrocellulose, ethyl cellulose, methylhydroxypropyl cellulose, hydroxyethyl cellulose, sodium cellulose sulfate, hydroxypropyl cellulose, sodium carboxymethyl cellulose, crystalline cellulose, and cellulose powder; and alginate polymers such as sodium alginate and propylene glycol alginate.

It further includes vinyl polymers such as polyvinyl methyl ether and carboxyvinyl polymer; polyoxyethylene polymers; polyoxyethylene/polyoxypropylene copolymers, acrylic polymers such as polyethyl acrylate and polyacrylamide; inorganic water-soluble polymers such as polyethyleneimine, cationic polymer, bentonite, magnesium aluminium silicate, laponite, hectorite, and silicic anhydride; PEG-240/decyltetradeceth-20/hexamethylene diisocyanate copolymer; (dimethylacrylamide/sodium acryloyldimethyltaurine) crosspolymer; (sodium acrylate/acryloyldimethyltaurine) copolymer; (alkyl acrylate/steareth-20 methacrylate) copolymer; and (ammonium acryloyldimethyltaurineNP) copolymer.

Among them, it is preferred that the water-soluble polymer as the component (e) is one or more selected from succinoglucan, polyacrylamide, carboxyvinyl polymer, and xanthan gum.

The amount of component (e) is 0.01 to 3.0 % by mass, preferably 0.03 to 2.0 % by mass, and more preferably 0.03 to 1.0 % by mass.

### ((f) Hydrophilic nonionic surfactant or hydrophilic anionic surfactant)

It is preferred that the oil-in-water external skin preparation according to the present invention further includes a hydrophilic nonionic surfactant or a hydrophilic anionic surfactant as the component (f). By incorporating a hydrophilic nonionic surfactant or a hydrophilic anionic surfactant, the emulsion stability can be further improved.

Examples of such hydrophilic nonionic surfactant or hydrophilic anionic surfactant are illustrated in the following.

Examples of hydrophilic nonionic surfactant used in the present invention include polyoxyethylene fatty acid ether, polyoxyethylene fatty acid ester, polyoxyethylene alkyl ether, polyoxyethylene polyoxypropylene alkyl ether, polyoxyethylene alkyl phenyl ether, polyoxyethylene cholesteryl ether, polyoxyethylene phytosterol ether, polyoxyethylene polyoxypropylene phytosterol ether, polyoxyethylene hydrogenated caster oil, polyoxyethylene sorbitan fatty acid ester, polyoxyethylene sorbit fatty acid ester, polyoxyethylene glyceryl fatty acid ester, polyglycerin fatty acid ester, and sucrose fatty acid ester.

Examples of hydrophilic anionic surfactant used in the present invention include fatty acid soap, N-acyl glutamate, acyl taurine salt, acyl alkyl taurine salt, higher alkyl sulfate ester salt, alkyl ether sulfate ester salt, N-acyl sarcosine acid salt, higher fatty acid amide sulfonate salt, phosphate ester salt, sulfosuccinic acid salt, and alkylbenzenesulfonate salts.

As aqueous phase components used in the present invention, depending on the object, various water-soluble components usually used in external skin emulsions can be incorporated within a quantitative/qualitative range that does not impair the effects of the present invention. Examples of lower alcohol include ethanol, propanol, and isopropanol.

Examples of moisturizer include polyethylene glycol, propylene glycol, dipropylene glycol, 1,3-butylene glycol, erythritol, threhalose, hexylene glycol, glycerin, diglycerin, polyglycerin, xylitol, maltitol, maltose, D-mannite, sorbitol, polyoxyethylene methyl gluceth, polyoxyethylene polyoxypropylene methyl gluceth, polyoxyethylene polyoxypropylene copolymer dimethyl ether, malt sugar syrup, glucose, fructose, sodium chondroitin sulfate, sodium hyaluronate, sodium adenosine phosphate, sodium lactate, bile acid salt, pyrrolidone carboxylic acid salt, glucosamine, cyclodextrin, and water-soluble collagen.

In the oil-in-water external skin preparation of the present invention, other components such as powder, oil-soluble UV absorbers, water-soluble UV absorbers, sequestering agents, neutralizers, pH adjusters, antioxidants, antibacterial agents, various drugs, and various extracts can be incorporated in addition to the above-mentioned components.

Examples of oil-soluble UV absorbers include p-aminobenzoic acid UV absorbers; anthranilic acid UV absorbers such as methyl anthranilate; salicylic acid UV absorbers such as octyl salicylate, phenyl salicylate, and homomethyl salicylate; and cinnamic acid UV absorbers such as isopropyl p-methoxycinnamate, octyl p-methoxycinnamate, 2-ethylhexyl p-methoxycinnamate, glyceryl mono-2-ethylhexanoate di-p-methoxycinnamate, and [4-bis(trimethylsiloxy)methylsilyl-3-methylbutyl]-3,4,5,-trimethoxy cinnamic acid ester.

Examples of water-soluble UV absorbers include benzophenone UV absorbers such as 2,4-dihydroxybenzophenone, 2-hydroxy-4-methoxybenzophenone, 2-hydroxy-4-methoxybenzophenone-5-sulfonic acid, and sodium 2-hydroxy-4-methoxybenzophenone-5-sulfonate; octyl p-methoxycinnamate; glyceryl octyl di-p-methoxycinnamate; urocanic acid; ethyl urocanate; 2-phenyl-5-methylbenzoxazole; 2-(2'-hydroxy-5'-methylphenyl)benzotriazole; and 4-tert-butyl-4'-methoxybenzoylmethane.

Examples of sequestering agents include sodium edetate, sodium polyphosphate, and sodium metaphosphate.

Examples of neutralizers include 2-amino-2-methyl-1-propanol, 2-amino-2-methyl-1,3-propanediol, potassium hydroxide, sodium hydroxide, triethanolamine, sodium carbonate, and amino acids.

Examples of pH adjusters include lactic acid, citric acid, glycolic acid, succinic acid, tartaric acid, dl-malic acid, potassium carbonate, sodium hydrogencarbonate, ammonium hydrogencarbonate, and phosphoric acid. Examples of antioxidants include ascorbic acid, alpha-tocopherol, dibutylhydroxytoluene, butylhydroxyanisole, sulfite, and pyrosulfite. Examples of antibacterial agents include benzoic acid, salicylic acid, sulfur, carbolic acid, sorbic acid, paraoxybenzoic acid ester, p-chloro-m-cresol, hexachlorophene, benzalkonium chloride, chlorhexidine chloride, trichlorocarbanilide, phenoxyethanol, parabens, and ethyl hexanediol.

Examples of various drugs include vitamins such as vitamin A oil, retinol, retinol palmitate, inositol, pyridoxine hydrochloride, benzyl nicotinate, nicotinamide, dl-alpha-tocopherol nicotinate, magnesium ascorbyl phosphate, sodium ascorbyl phosphate, vitamin D2 (ergocalciferol), dl-alpha-tocopherol, dl-alpha-tocopheryl acetate, pantothenic acid, and biotin; amino acids such as arginine, aspartic acid, cystine, cysteine, methionine, serine, leucine, tryptophan, alanine, glycine, and trimethylglycine; anti-inflammatory agents such as allantoin, glycyrrhizinic acid, glycyrrhetinic acid, and azulene; whitening agents such as alkoxysalicylic acid, tranexamic acid, linoleic acid, ascorbic acid glucoside, ethyl vitamin C, and arbutin; astringent agents such as zinc oxide and tannic acid; refresheners such as L-menthol and camphor; sulfur; lysozyme chloride; and gamma-orizanol.

Examples of various extracts include houttuynia cordata extract, amur corktree bark extract, scutellaria baicalensis root extract, coptis extract, melilotus officinalis extract, nettle extract, licorice extract, paeonia lactiflora extract, soapwort extract, loofah extract, saxifraga stolonifera extract, sophora extract, fennel extract, rose extract, rehmannia glutinosa extract, lemon extract, turmeric extract, aloe extract, calamus root extract, eucalyptus extract, field horsetail extract, sage leaf extract, thyme extract, tea extract, seaweed extract, cucumber extract, clove extract, raspberry extract, lemon balm extract, carrot extract, horse chestnut extract, peach extract, peach leaf extract, mulberry extract, cornflower extract, ginger extract, yuzu (Citrus junos) extract, yuzu (Citrus junos) seed extract, hamamelis extraction liquid, and silk extraction liquid.

The above-mentioned water-soluble drugs can be used in a free state, a form of acid or basic salt if one can become salts, or a form of ester if one has a carbonic acid group. In the oil-in-water external skin preparation of the present invention, as necessary, appropriate perfumes, coloring agents, and pigments can be further added within a range that does not deteriorate the emulsion stability.

The oil-in-water external skin preparation according to the present invention has thread-forming property, and, as mentioned above, the length of formed thread at a velocity of 30 cm/min. is preferably 20 mm or more to 200 mm or less. Such oil-in-water external skin preparation of the present invention can be produced in a normal method. However, the thread-forming property and the length of formed thread which are obtained by the sodium polyacrylate may be varied owing to the physical shearing such as a homogenizer. When a preferable length of formed thread cannot be obtained, though it depends on the amount of each component, the sodium polyacrylate may be added and dissolved after the completion of dissolving and emulsifying the components other than the sodium polyacrylate or other than polyacrylic acid or a metal salt thereof.

The viscosity of the oil-in-water external skin preparation according to the present invention is preferably 3000 to 50000 mPa•s as the viscosity just after the production, and more preferably 5000 to 30000 mPa•s.

The oil-in-water external skin preparation of the present invention can take any of various product forms without a particular limitation, and the specific examples include liquid, milky liquid, and cream form products such as essence, milky lotion, cream, milky lotion for daytime use, pre-makeup, liquid foundation, eyeliner, mascara, and hair gel. Particularly, essence is preferred.

### EXAMPLES

Hereinafter, the present invention will be further illustrated in the following examples. However, these examples should not limit the present invention in any manner. Unless otherwise noted, the amount of each component is expressed in mass %.

Prior to illustrating the examples, the evaluation methods for the tests used in the present invention will be explained.

### Evaluation (1): Thread-forming property

Whether a sample just after the production had thread-forming property when applied to skin was evaluated in accordance with the following evaluation method.
- Evaluation method
   O: It has thread-forming property.
   X: It does not have thread-forming property.

### Evaluation (2): Richness

20 professional panelists conducted sensory test by applying each of the samples just after the production to skin and evaluated the richness. The rating criteria is as follows.

### Rating criteria

5 points: Highly richness
4 points: Slightly richness
3 points: Normal
2 points: Lacking richness slightly
1 point: No richness at all

### Evaluation (3): Fresh feeling

20 professional panelists conducted sensory test by applying each of the samples just after the production to skin and evaluated the fresh feeling. The rating criteria is as follows.

### Rating criteria

5 points: Highly fresh feeling
4 points: Slightly fresh feeling
3 points: Normal
2 points: Lacking fresh feeling slightly
1 point: No fresh feeling at all

### Evaluation (4): Non-stickiness

20 professional panelists conducted sensory test by applying each of the samples just after the production to skin and evaluated the stickiness when the applied sample got dry. The rating criteria is as follows.

### Rating criteria

5 points: No stickiness at all
4 points: Devoid of stickiness slightly
3 points: Normal
2 points: Slightly sticky
1 point: Highly sticky

### Evaluation (5): Time-dependent stability of thread-forming property

The lengths of formed threads of each sample just after the production (A) and of each sample which had been preserved in a thermostatic bath at 50°C for 1 month (B) were measured. The time-dependent stability of thread-forming property was evaluated in accordance with the following evaluation method.
- Evaluation method
   ⊚: (B)/(A) = 0.8 or more
   ○: (B)/(A) = 0.6 or more to less than 0.8
   Δ: (B)/(A) = 0.4 or more to less than 0.6
   X: (B)/(A) = less than 0.4

Here, when the length of formed thread of a sample just after the production was less than 20 mm, the sample was evaluated as "X".

### Evaluation (6): Length of formed thread

The surface of each sample just after the production was adjusted to be 30 °C and flatted. The surface of the sample was lightly contacted with a stainless-steel round disk having a diameter of about 1 cm uniformly, then the sample was dropped vertically at a velocity of 30 cm/min.. The state of forming threads of the sample was observed to measure the length of thread when the thread breaks.

### Evaluation (7): Viscosity

The viscosity of each sample just after the production was measured at a rotation speed of 12 rpm for 1 minute with a VDA-type viscometer (DIGITAL VISMETRON VDA of Shibaura Systems Co., LTD.) using a rotor No. 3 or No. 4.

### Evaluation (8): Penetration into skin

20 professional panelists conducted sensory test by applying each of the samples just after the production to skin and evaluated the penetration into skin. The rating criteria is as follows.

### Rating criteria

5 points: It penetrates into skin very well.
4 points: It penetrates into skin slightly well.
3 points: It penetrates into skin normally.
2 points: It penetrates into skin slightly poorly.
1 point: It penetrates into skin very poorly.

Firstly, the present inventors produced each sample with the blending composition shown in Table 1 in which any of various components were incorporated as a water-soluble polymer.

Then, each sample was evaluated for the evaluation item (1) in the above-mentioned evaluation method, and it was measured and evaluated for the evaluation item (5) in the above-mentioned method. Also, the professional panelists evaluated each sample for the evaluation items (2) to (4) according to the above-mentioned rating criteria, and the total score was evaluated in accordance with the following professional panelist evaluation method. The result is shown in Table 1.
- Professional panelist evaluation method
   ⊚: The total score is 80 or more.
   O: The total score is 60 or more to less than 80.
   Δ: The total score is 40 or more to less than 60.
   X: The total score is less than 40.

**Table 1**

| | 1-1 | 1-2 | 1-3 | 1-4 |
|---|---|---|---|---|
| Sodium polyacrylate | 1 | - | - | - |
| Hyaluronic acid | - | 1 | - | - |
| Xanthan gum | - | - | 1 | - |
| Glycerin | - | - | - | 1 |
| Water | 99 | 99 | 99 | 99 |
| (1) Thread-forming property | O | O | O | X |
| (2) Richness | ⊚ | O | O | X |
| (3) Fresh feeling | O | Δ | Δ | O |
| (4) Non-stickiness | Δ | Δ | X | ⊚ |
| (5) Time-dependent stability of thread-forming property | X | O | O | X |

Test Example 1-1, in which sodium polyacrylate was incorporated as water-soluble polymer, had thread-forming property and provided a very significant richness and a slightly excellent fresh feeling, but it provided a slight stickiness and had a poor time-dependent stability of the thread-forming property.

Test Example 1-2, in which hyaluronic acid was incorporated, had thread-forming property and was slightly excellent in richness and time-dependent stability of the thread-forming property, but it was slightly poor in a fresh feeling and non-stickiness.

Test Example 1-3, in which xanthan gum was incorporated, had thread-forming property and was slightly excellent in richness and time-dependent stability of the thread-forming property, but it was slightly poor in a fresh feeling and provided stickiness so much.

Test Example 1-4, in which glycerin was incorporated, provided no stickiness at all and was slightly excellent in a fresh feeling, but it had no thread-forming property and provided no richness at all.

From the above, it became apparent that, when sodium polyacrylate is incorporated as a water-soluble polymer, the preparation can have thread-forming property and also provide a very significant richness, compared with the compositions wherein any of other water-soluble polymers is incorporated. However, it also became apparent that such preparation provides a slight stickiness and has a serious problem in the time-dependent stability of thread-forming property.

Next, the present inventors studied the kinds of other components necessary for obtaining the preparation which provides the fresh feeling and no stickiness and has time-dependent stability of the thread-forming property as well as which incorporates sodium polyacrylate to provide a highly excellent richness.

They prepared each sample with the blending composition shown in Table 2. Test Examples 2-1 to 2-4 were prepared in a normal method. In Test Examples 2-5 to 2-9, each oil-in-water external skin preparation (essence) was prepared according to the following production method.

### Production method of essence

The aqueous phase other than sodium polyacrylate was mixed, and the oil phase was gradually added thereto. After the completion of emulsifying these components, sodium polyacrylate was added and dissolved in the emulsion, and the mixture was stirred with a homogenizer.

The professional panelists evaluated each sample for the evaluation items (2) to (4) according to the above-mentioned rating criteria, and the total score was evaluated in accordance with the above-mentioned professional panelist evaluation method. Also, each sample was measured and evaluated for the evaluation item (5) in the above-mentioned method. The result is shown in Table 2.

**Table 2**

| | 2-1 | 2-2 | 2-3 | 2-4 | 2-5 | 2-6 | 2-7 | 2-8 | 2-9 |
|---|---|---|---|---|---|---|---|---|---|
| Sodium polyacrylate *1 | 1 | 1 | 1 | 1 | 1 | 1 | 1 | 1 | 1 |
| (C10 to C30) Acrylic acid/alkyl methacrylate copolymer *2 | 0.5 | 0.5 | - | - | 0.5 | 0.5 | - | - | - |
| Carboxyvinyl polymer | - | - | 0.5 | 0.5 | - | - | 0.5 | 0.5 | - |
| Polyethylene glycol 6000 | 2 | - | 2 | - | 2 | - | 2 | - | - |
| Dextrin | - | 2 | - | 2 | - | 2 | - | 2 | - |
| Water | 96.5 | 96.5 | 96.5 | 96.5 | 91.3 | 91.3 | 91.3 | 91.3 | 93.8 |
| Polyoxyethylene phytosterol ether | - | - | - | - | 0.2 | 0.2 | 0.2 | 0.2 | 0.2 |
| Squalane | - | - | - | - | 5 | 5 | 5 | 5 | 5 |
| (2) Richness | ⊚ | ⊚ | ⊚ | ⊚ | ⊚ | ⊚ | ⊚ | ⊚ | ⊚ |
| (3) Fresh feeling | Δ | Δ | Δ | Δ | ⊚ | ⊚ | Δ | Δ | X |
| (4) Non-stickiness | Δ | X | X | X | ⊚ | O | Δ | Δ | Δ |
| (5) Time-dependent stability of thread-forming property | ⊚ | Δ | O | Δ | ⊚ | Δ | O | Δ | X |

| | | | | | | | | | |
|---|---|---|---|---|---|---|---|---|---|
| *1: ARONBIS S, manufactured by Nihon Junyaku Co., Ltd. *2: Pemulen TR-2, manufactured by Lubrizol Advanced Materials, Inc. | | | | | | | | | |

Test Example 2-1, in which alkyl-modified carboxyvinyl polymer and polyethylene glycol were incorporated with sodium polyacrylate, provided an excellent richness and had time-dependent stability of thread-forming property, but it was slightly poor in a fresh feeling and non-stickiness.

Test Example 2-2, in which alkyl-modified carboxyvinyl polymer and dextrin were incorporated with sodium polyacrylate, provided an excellent richness, but it was slightly poor in a fresh feeling and time-dependent stability of the thread-forming property and provided stickiness so much.

Test Example 2-3, in which carboxyvinyl polymer and polyethylene glycol were incorporated with sodium polyacrylate, provided an excellent richness and had a slightly good time-dependent stability of the thread-forming property, but it was slightly poor in a fresh feeling and provided stickiness so much.

Test Example 2-4, in which carboxyvinyl polymer and dextrin were incorporated with sodium polyacrylate, provided an excellent richness, but it was slightly poor in a fresh feeling and time-dependent stability of the thread-forming property and provided stickiness so much.

Test Example 2-5, which was an oil-in-water external skin preparation prepared by incorporating oils in Test Example 2-1, provided an excellent richness, had time-dependent stability of the thread-forming property, and was highly excellent in the usability such as a fresh feeling and non-stickiness when the applied preparation got dry.

Test Example 2-6, which was an oil-in-water external skin preparation prepared by incorporating oils in Test Example 2-2, was excellent in richness and a fresh feeling and was slightly excellent in non-stickiness, but it was slightly poor in time-dependent stability of the thread-forming property.

Test Example 2-7, which was an oil-in-water external skin preparation prepared by incorporating oils in Test Example 2-3, was excellent in richness and slightly good in time-dependent stability of the thread-forming property, but it was slightly poor in the usability such as a fresh feeling and non-stickiness.

Test Example 2-8, which was an oil-in-water external skin preparation prepared by incorporating oils in Test Example 2-4, was excellent in richness, but the usability such as a fresh feeling and non-stickiness was not sufficient, and the thread-forming property was not stable slightly over time.

Test Example 2-9, in which sodium polyacrylate was incorporated in an oil-in-water external skin preparation, was excellent in richness, but it was slightly poor in non-stickiness and poor in a fresh feeling and time-dependent stability of the thread-forming property.

From the above, it became apparent that the external skin preparation ,wherein it has thread-forming property and time-dependent stability of the thread-forming property and provides a highly excellent richness, a fresh feeling, and no stickiness, can be obtained by preparing the oil-in-water external skin preparation by incorporating sodium polyacrylate (a), alkyl-modified carboxyvinyl polymer (b) and polyethylene glycol (c), and further incorporating oil (d) therein.

In the oil-in-water external skin preparation incorporating the above-mentioned components (a) to (d), the present inventors studied the amount of these components and the kind of other components in detail.

They prepared each sample with the blending composition shown in Tables 3 and 4 and produced each oil-in-water external skin preparation (essence) according to the following production method.

### Production method of essence

### <Test Examples 3-1 to 3-11, Test Examples 4-1 to 4-6>

Each essence with the composition shown in Tables 3 and 4 was prepared in a normal method (i.e., after the production of aqueous phase, the oil phase was gradually added thereto). But here, the length of formed thread which can be obtained by sodium polyacrylate will be easily shortened by physical stirrer such as a homogenizer. Thus, the desired length of formed thread was obtained by dissolving and emulsifying the components other than sodium polyacrylate, then adding and dissolving sodium polyacrylate therein, and stirring the mixture with a homogenizer.

### <Test Example 4-7>

The essence with the composition in Test Example 4-7 shown in Table 4 was prepared in a normal method as mentioned above.

The professional panelists evaluated each sample for the evaluation items (3), (4), and (8) according to the above-mentioned rating criteria, and the total score was evaluated in accordance with the above-mentioned professional panelist evaluation method. Also, each sample was measured and evaluated for the evaluation item (5) in the above-mentioned method, and it was also measured for the evaluation items (6) to (7) in the above-mentioned method. The result is shown in Tables 3 and 4.

**Table 3**

| | 3-1 | 3-2 | 3-3 | 3-4 | 3-5 | 3-6 | 3-7 | 3-8 | 3-9 | 3-10 | 3-11 |
|---|---|---|---|---|---|---|---|---|---|---|---|
| Sodium polyacrylate *1 | 0.03 | 0.1 | 0.15 | 0.2 | 0.8 | 1 | 2 | 3 | 2 | 0.15 | 2 |
| (C10 to C30) Acrylic acid/alkyl methacrylate copolymer *2 | 0.2 | 0.05 | 0.1 | 0.2 | 0.3 | 0.2 | 1 | 0.2 | 0.5 | 0.1 | 0.5 |
| Polyethylene glycol 1500 | - | - | - | 5 | - | - | - | - | - | - | - |
| Polyethylene glycol 6000 | 8 | 3 | 1 | - | - | 10 | 3 | 5 | 2 | 1 | 2 |
| Polyethylene glycol 20000 | - | - | - | - | 4 | - | - | - | - | - | - |
| Squalane | 4 | 1 | - | - | 2 | 3 | 3 | 1 | 5 | - | 5 |
| Dimethylpolysiloxane | - | 2 | - | 1 | 2 | 1 | 8 | - | 1 | - | 1 |
| Glyceryl triethylhexanoate | 4 | - | 5 | 2 | 2 | 1 | 7 | 1 | 2 | 5 | 2 |
| Carboxyvinyl polymer | 0.3 | 0.2 | 0.1 | 0.4 | 1 | - | 0.6 | 0.4 | - | 0.1 | - |
| Succinoglucan | 0.05 | 0.2 | 0.4 | 0.1 | - | 0.7 | 0.3 | 0.3 | - | 0.4 | - |
| Polyoxyethylene hydrogenated caster oil | 0.05 | 0.05 | 0.05 | 0.05 | 0.05 | 0.05 | 0.05 | 0.05 | 0.05 | - | - |
| Sodium hydroxide | Q.S. | Q.S. | Q.S. | Q.S. | Q.S. | Q.S. | Q.S. | Q.S. | Q.S. | Q.S. | Q.S. |
| Tranexamic acid | 1 | 3 | 4 | 2 | 4 | 1 | 1 | 3 | 1 | 4 | 1 |
| Ethyl alcohol | 5 | 3 | 6 | - 10 2 | 10 | 2 | 5 | 3 | - | 6 | - |
| Methyl paraben | Q.S. | Q.S. | Q.S. | Q.S. | Q.S. | Q.S. | Q.S. | Q.S. | Q.S. | Q.S. | Q.S. |
| Edetate | Q.S. | Q.S. | Q.S. | Q.S. | Q.S. | Q.S. | Q.S. | Q.S. | Q.S. | Q.S. | Q.S. |
| Perfume | Q.S. | Q.S. | Q.S. | Q.S. | Q.S. | Q.S. | Q.S. | Q.S. | Q.S. | Q.S. | Q.S. |
| Water | Balance | Balance | Balance | Balance | Balance | Balance | Balance | Balance | Balance | Balance | Balance |
| Total | 100 | 100 | 100 | 100 | 100 | 100 | 100 | 100 | 100 | 100 | 100 |
| (3) Fresh feeling | ⊚ | ⊚ | ⊚ | ⊚ | ⊚ | ⊚ | O | ⊚ | O | ⊚ | O |
| (4) Non-stickiness | ⊚ | ⊚ | ⊚ | ⊚ | ⊚ | O | O | O | O | ⊚ | O |
| (5) Time-dependent stability of thread-forming property | ⊚ | ⊚ | ⊚ | ⊚ | ⊚ | ⊚ | ⊚ | ⊚ | ⊚ | ⊚ | ⊚ |
| (6) Length of formed thread (mm) | 25 | 40 | 60 | 70 | 110 | 130 | 150 | 170 | 120 | 65 | 115 |
| (7) Viscosity (mPa·s) | 19000 | 12000 | 10000 | 30000 | 48000 | 15000 | 40000 | 38000 | 7000 | 11500 | 6500 |
| (8) Penetration into skin | ⊚ | ⊚ | ⊚ | ⊚ | ⊚ | O | O | O | ⊚ | ⊚ | ⊚ |

**Table 4**

| | 4-1 | 4-2 | 4-3 | 4-4 | 4-5 | 4-6 | 4-7 |
|---|---|---|---|---|---|---|---|
| Sodium polyacrylate *1 | - | 0.15 | 0.03 | 0.03 | 0.03 | 0.05 | 0.1 |
| (C10 to C30) Acrylic acid/alkyl methacrylate copolymer *2 | 0.15 | - | 0.04 | 0.2 | 0.05 | 0.2 | 0.05 |
| Polyethylene glycol 6000 | 3 | 1 | - | - | - | 3 | 3 |
| Polyethylene glycol 400 | - | - | - | 5 | - | - | - |
| Highly polymerized polyethylene glycol (molecular weight 200000) | - | - | - | - | 5 | - | - |
| Squalane | - | - | 4 | 7 | - | - | 1 |
| Dimethylpolysiloxane | 3 | 5 | 1 | - | 4 | - | 2 |
| Glyceryl triethylhexanoate | 1 | 2 | 5 | - | - | - | - |
| Carboxyvinyl polymer | - | - | 0.4 | 0.6 | 0.4 | 0.5 | 0.2 |
| Succinoglucan | 0.3 | 1.0 | 0.5 | - | - | 0.1 | 0.2 |
| Polyoxyethylene hydrogenated caster oil | 0.05 | 0.5 | 0.05 | 0.05 | 0.05 | 0.05 | 0.05 |
| Sodium hydroxide | Q.S. | Q.S. | Q.S. | Q.S. | Q.S. | Q.S. | Q.S. |
| Tranexamic acid | 1 | 3 | 1 | 1 | 1 | 2 | 3 |
| Ethyl alcohol | 2 | 7 | 3 | 5 | 8 | 6 | 3 |
| Methyl paraben | Q.S. | Q.S. | Q.S. | Q.S. | Q.S. | Q.S. | Q.S. |
| Edetate | Q.S. | Q.S. | Q.S. | Q.S. | Q.S. | Q.S. | Q.S. |
| Perfume | Q.S. | Q.S. | Q.S. | Q.S. | Q.S. | Q.S. | Q.S. |
| Water | Balance | Balance | Balance | Balance | Balance | Balance | Balance |
| Total | 100 | 100 | 100 | 100 | 100 | 100 | 100 |
| (3) Fresh feeling | Δ | Δ | ⊚ | ⊚ | Δ | Δ | ⊚ |
| (4) Non-stickiness | ⊚ | Δ | O | ⊚ | X | X | ⊚ |
| (5) Time-dependent stability of thread-forming property | X | X | Δ | Δ | ⊚ | ⊚ | X |
| (6) Length of formed thread (mm) | 10 | 50 | 30 | 30 | 30 | 30 | 15 |
| (7) Viscosity (mPa·s) | 5000 | 12000 | 25400 | 34000 | 21800 | 24500 | 11500 |
| (8) Penetration into skin | ⊚ | X | ⊚ | O | X | ⊚ | ⊚ |

Each of Test Examples 3-1 to 3-11, in which sodium polyacrylate, alkyl-modified carboxyvinyl polymer, and polyethylene glycol having an average molecular weight of 1500, 6000, or 20000 were appropriately incorporated, had an adequate viscosity, an adequate length of formed thread, time-dependent stability of the thread-forming property and an excellent usability such as a fresh feeling, penetration into skin, and non-stickiness.

On the other hand, Test Example 4-1 incorporating no sodium polyacrylate was excellent in penetration into skin and non-stickiness, but it was poor in the length of formed thread and slightly poor in a fresh feeling.

Test Example 4-2 incorporating no alkyl-modified carboxyvinyl polymer had an adequate length of formed thread, but it was poor in time-dependent stability of the thread-forming property, and there were serious problems in the usability.

Test Example 4-3 incorporating no polyethylene glycol had a good usability, but the thread-forming property became slightly poor over time.

Test Example 4-4 incorporating polyethylene glycol having an average molecular weight of 400 was excellent in the usability, but it was slightly poor in time-dependent stability of the thread-forming property.

Test Example 4-5 incorporating highly-polymerized polyethylene glycol having an average molecular weight of 200000 had a very stable thread-forming property over time, but there were serious problems in the usability.

Test Example 4-6 incorporating sodium polyacrylate, alkyl-modified carboxyvinyl polymer, and polyethylene glycol having an average molecular weight of 6000, but no oils, could not form an oil-in-water external skin preparation, and it was slightly poor in a fresh feeling and poor in non-stickiness though it was excellent in penetration into skin and time-dependent stability of the thread-forming property.

Test Example 4-7 incorporating sodium polyacrylate, alkyl-modified carboxyvinyl polymer, and polyethylene glycol having an average molecular weight of 6000, which were same components as Test Example 3-2, but not adding and dissolving sodium polyacrylate after the completion of emulsification, was excellent in a fresh feeling, penetration into skin, and non-stickiness, but it was poor in the length of formed thread and very poor in richness.

As a result that the present inventors further studied in detail, they found that the oil-in-water external skin preparation ,wherein it has an adequate length of formed thread and is excellent in the usability and the time-dependent stability of thread-forming property, can be obtained by incorporating (a) 0.01 to 3.0 % by mass of sodium polyacrylate, (b) 0.01 to 1.0 % by mass of alkyl-modified carboxyvinyl polymer, (c) 1.0 to 10.0 % by mass of polyethylene glycol having an average molecular weight of 1000 to 50000, and (d) 0.5 to 20.0 % by mass of oil.

Hereinafter, formulation examples of the oil-in-water external skin preparation of the present invention will be illustrated. It is to be understood that the present invention is not limited by these formulation examples in any manner and is specified by the scope of claims. Each of the obtained oil-in-water external skin preparations had thread-forming property and an excellent time-dependent stability of the thread-forming property, provided richness, a fresh feeling, and no stickiness when the applied preparation got dry, and penetrated well into skin.

### Formulation Example 1 Essence

| | |
|---|---|
| Sodium polyacrylate | 0.3 % by mass |
| (C10 to C30) acrylic acid/alkyl methacrylate copolymer | 0.1 |
| Polyethylene glycol 1500 | 3.0 |
| Cetyl octanoate | 1.0 |
| Decamethylcyclopentasiloxane | 2.0 |
| Squalane | 2.0 |
| Pentaerythritol tetraoctanoate | 1.0 |
| Polyacrylamide | 0.1 |
| (Sodium acrylate/acryloyl dimethyl taurine) copolymer | 0.1 |
| Sodium stearoyl methyl taurate | 0.1 |
| Ethanol | 5.0 |
| Glycerin | 6.0 |
| 1,3-Butylene glycol | 5.0 |
| PEG/PPG-17/4 dimethyl ether | 5.0 |
| Tranexamic acid methylamide hydrochloride | 2.0 |
| Dipotassium glycyrrhizinate | 0.05 |
| Tocopheryl acetate | 0.2 |
| Xanthan gum | 0.1 |
| Silica | 2.0 |
| Polyethylene powder | 1.0 |
| Sodium metaphosphate | Q.S. |
| Potassium hydroxide | Q.S. |
| Citric acid | Q.S. |
| Sodium citrate | Q.S. |
| Yellow iron oxide | Q.S. |
| Phenoxyethanol | Q.S. |
| Perfume | Q.S. |
| Purified water | Balance |

### (Production method)

After the aqueous phase was prepared, the oil phase was gradually added thereto. The mixture was emulsified with a homogenizer, and then sodium polyacrylate was dissolved therein.

### Formulation Example 2 Essence

| Sodium polyacrylate | 0.1 % by mass |
|---|---|
| (C10 to C30) acrylic acid/alkyl methacrylate copolymer | 0.1 |
| Polyethylene glycol 1000 | 2.0 |
| Polyethylene glycol 20000 | 1.0 |
| Decamethylcyclopentasiloxane | 2.0 |
| Di(phytosteryl/octyldodecyl) lauroyl glutamate | 0.5 |
| Ethanol | 5.0 |
| Glycerin | 2.0 |
| PEG/PPG-17/4 dimethyl ether | 10.0 |
| Sodium hyaluronate | 0.3 |
| Ethyl vitamin C | 2.0 |
| Salicylic acid | 0.5 |
| Dipotassium glycyrrhizinate | 0.01 |
| Nicotinamide | 3.0 |
| Sodium metaphosphate | Q.S. |
| Potassium hydroxide | Q.S. |
| Citric acid | Q.S. |
| Sodium citrate | Q.S. |
| Phenoxyethanol | Q.S. |
| Perfume | Q.S. |
| Purified water | Balance |

### (Production method)

After the aqueous phase was prepared, the oil phase was gradually added thereto. The mixture was emulsified with a homogenizer, and then sodium polyacrylate was dissolved therein.

### Formulation Example 3 Milky lotion

| Sodium polyacrylate | 0.8 % by mass |
|---|---|
| (C10 to C30) acrylic acid/alkyl methacrylate copolymer *3 | 0.3 |
| Polyethylene glycol 11000 | 4.0 |
| Glyceryl triethylhexanoate | 5.0 |
| Xanthan gum | 0.3 |
| (Dimethylacrylamide/sodium acryloyl dimethyl taurine) crosspolymer | 0.8 |
| Polyoxyethylene hydrogenated caster oil | 1.0 |
| Polyoxyethylene phytosterol ether | 0.3 |
| Dipropylene glycol | 10.0 |
| Maltitol | 2.0 |
| Sorbitol | 4.0 |
| Xylitol | 0.5 |
| Sodium metaphosphate | Q.S. |
| Potassium hydroxide | Q.S. |
| Phenoxyethanol | Q.S. |
| Purified water | Balance |

| | |
|---|---|
| *3: Pemulen TR-1, manufactured by Lubrizol Advanced Materials, Inc. | |

### (Production method)

After the aqueous phase was prepared, the oil phase was gradually added thereto. The mixture was emulsified with a homogenizer, and then sodium polyacrylate was dissolved therein.

### Formulation Example 4 Cream

| Sodium polyacrylate | 0.1 % by mass |
|---|---|
| (C10 to C30) acrylic acid/alkyl methacrylate copolymer *4 | 0.1 |
| Polyethylene glycol 6000 | 2.0 |
| Agar | 1.0 |
| Hydrogenated polyisobutene | 2.0 |
| Decamethylcyclopentasiloxane | 5.0 |
| Tridecyl isononanoate | 1.0 |
| Batyl alcohol | 2.0 |
| Petrolatum | 1.0 |
| (Ammonium acryloyl dimethyl taurine/VP) copolymer | 1.5 |
| POE(30) behenyl ether | 3.0 |
| Sorbitan monooleate | 0.5 |
| Myristyl myristate | 1.5 |
| 1,3-Butylene glycol | 3.0 |
| Glycerin | 2.0 |
| Ethyl vitamin C | 0.5 |
| Magnesium ascorbate phosphate ester | 0.5 |
| Potassium hydroxide | Q.S. |
| Citric acid | Q.S. |
| Sodium citrate | Q.S. |
| Methyl paraben | Q.S. |
| Perfume | Q.S. |
| Purified water | Balance |

| | |
|---|---|
| *4: Carbopol 1342, manufactured by Lubrizol Advanced Materials, Inc. | |

### (Production method)

After the aqueous phase was prepared and heated, the heated oil phase was gradually added thereto. The mixture was emulsified with a homogenizer, and then sodium polyacrylate was dissolved therein.

### Formulation Example 5 Milky lotion for daytime use

| Sodium polyacrylate | 0.05 % by mass |
|---|---|
| (C10 to C30) acrylic acid/alkyl methacrylate copolymer | 0.4 |
| Polyethylene glycol 1000 | 5.0 |
| Myristyl myristate | 3.0 |
| 2-Ethylhexyl 2-ethylhexanoate | 2.0 |
| Dimethylpolysiloxane | 2.0 |
| Petrolatum | 0.5 |
| Behenyl alcohol | 2.0 |
| Carboxyvinyl polymer | 0.5 |
| PEG(40) glyceryl isostearate | 1.2 |
| Sorbitan stearate | 0.3 |
| Stearic acid | 0.5 |
| Diglycerin | 10.0 |
| 1,3-Butylene glycol | 1.0 |
| Erythritol | 2.0 |
| Octyl p-methoxycinnamate | 6.0 |
| Glyceryl octyl di-p-methoxycinnamate | 2.0 |
| 4-tert-butyl-4'-methoxydibenzoylmethane | 1.0 |
| Potassium 4-methoxysalicylate | 2.0 |
| Sodium hydroxide | Q.S. |
| Methyl paraben | Q.S. |
| Perfume | Q.S. |
| Purified water | Balance |

### (Production method)

After the aqueous phase was prepared, the oil phase was gradually added thereto. The mixture was emulsified with a homogenizer, and then sodium polyacrylate was dissolved therein.

### Formulation Example 6 Pre-makeup

| Sodium polyacrylate | 0.2 % by mass |
|---|---|
| (C10 to C30) acrylic acid/alkyl methacrylate copolymer | 0.3 |
| Polyethylene glycol 1500 | 4.0 |
| Dimethylpolysiloxane | 2.0 |
| Decamethylcyclopentasiloxane | 5.0 |
| Behenyl alcohol | 0.5 |
| Batyl alcohol | 0.3 |
| Polyoxyethylene glyceryl isostearate | 0.3 |
| Dipropylene glycol | 5.0 |
| 1,3-Butylene glycol | 3.0 |
| Tocopheryl acetate | 0.1 |
| Titanium oxide/red iron oxide coated mica | 0.3 |
| Titanated mica | 0.3 |
| Porous spherical cellulose powder | 0.5 |
| Sodium metaphosphate | Q.S. |
| Potassium hydroxide | Q.S. |
| Methyl paraben | Q.S. |
| Perfume | Q.S. |
| Purified water | Balance |

### (Production method)

After the aqueous phase was prepared, the oil phase was gradually added thereto. The mixture was emulsified with a homogenizer, and then sodium polyacrylate was dissolved therein.

### Formulation Example 7 Liquid foundation

| Sodium polyacrylate | 0.3 % by mass |
|---|---|
| (C10 to C30) acrylic acid/alkyl methacrylate copolymer | 0.3 |
| Polyethylene glycol 20000 | 3.0 |
| Dimethylpolysiloxane | 6.0 |
| Behenyl alcohol | 0.3 |
| Batyl alcohol | 0.7 |
| 1,3-Butylene glycol | 3.0 |
| Isostearic acid | 1.4 |
| Stearic acid | 1.0 |
| Behenic acid | 0.7 |
| Cetyl ethylhexanoate | 2.0 |
| Polyoxyethylene glyceryl monostearate | 2.0 |
| Glyceryl monostearate (self-emulsification) | 0.5 |
| Yellow iron oxide coated titanated mica | 2.0 |
| Titanium oxide | 4.0 |
| Talc | 0.5 |
| Kaolin | 3.0 |
| Synthetic phlogopite | 0.1 |
| Cross-linked silicone powder | 0.3 |
| Silicic anhydride | 5.0 |
| Tocopheryl acetate | 0.1 |
| Sodium hyaluronate | 0.1 |
| p-Hydroxybenzoate ester | Q.S. |
| 2-Ethylhexyl p-methoxycinnamate | 1.0 |
| Red iron oxide | Q.S. |
| Yellow iron oxide | Q.S. |
| Black iron oxide | Q.S. |
| Triethanolamine | Q.S. |
| Methyl paraben | Q.S. |
| Perfume | Q.S. |
| Purified water | Balance |

### (Production method)

After the aqueous phase was prepared, the oil phase was gradually added thereto. The mixture was emulsified with a homogenizer, and then sodium polyacrylate was dissolved therein.

### Formulation Example 8 Mascara

| Sodium polyacrylate | 1.0 % by mass |
|---|---|
| (C10 to C30) acrylic acid/alkyl methacrylate copolymer | 0.2 |
| Polyethylene glycol 11000 | 1.0 |
| Decamethylcyclopentasiloxane | 2.0 |
| Xanthan gum | 1.0 |
| Sodium carboxymethylcellulose | 1.0 |
| Polyoxyethylene hydrogenated caster oil | 0.2 |
| Ethanol | 10.0 |
| Glycerin | 1.0 |
| Silicic anhydride | 1.0 |
| Red iron oxide coated titanated mica (pearlescent agent) | 8.0 |
| Black iron oxide | 1.0 |
| Sodium metaphosphate | Q.S. |
| Sodium hydroxide | Q.S. |
| p-Hydroxybenzoate ester | Q.S. |
| Phenoxyethanol | Q.S. |
| Perfume | Q.S. |
| Purified water | Balance |

### (Production method)

After the aqueous phase was prepared, the oil phase was gradually added thereto. The mixture was emulsified with a homogenizer, and then sodium polyacrylate was dissolved therein.

### Formulation Example 9 Hair gel

| Sodium polyacrylate | 0.5 % by mass |
|---|---|
| (C10 to C30) acrylic acid/alkyl methacrylate copolymer | 0.5 |
| Polyethylene glycol 1500 | 5.0 |
| Liquid paraffin | 1.0 |
| Dimethylpolysiloxane | 1.0 |
| Highly polymerized methylpolysiloxane | 4.0 |
| Hydroxypropyl cellulose | 0.4 |
| Ethyl cellulose | 0.3 |
| Polyvinylpyrrolidone | 0.5 |
| Polyoxyethylene glyceryl fatty acid ester | 0.2 |
| 1,3-Butylene glycol | 5.0 |
| Ethanol | 20.0 |
| Sodium edetate | Q.S. |
| 2-Amino-2-methyl-1,3-propanediol | Q.S. |
| Methyl paraben | Q.S. |
| Perfume | Q.S. |
| Purified water | Balance |

### (Production method)

After the aqueous phase was prepared, the oil phase was gradually added thereto. The mixture was emulsified with a homogenizer, and then sodium polyacrylate was dissolved therein.

## Claims

1. An oil-in-water external skin preparation comprising following components (a) to (d):
(a) 0.01 to 3.0 % by mass of sodium polyacrylate;
(b) 0.01 to 1.0 % by mass of alkyl-modified carboxyvinyl polymer;
(c) 1.0 to 10.0 % by mass of polyethylene glycol having an average molecular weight of 1000 or more to 50000 or less; and
(d) 0.5 to 20.0 % by mass of oil; and
wherein the oil-in-water external skin preparation has thread-forming property.

2. The oil-in-water external skin preparation according to claim 1, further comprising a component (e): a water-soluble polymer other than a polyacrylic acid or metal salt thereof and other than component (b).

3. The oil-in-water external skin preparation according to claim 1 or 2, wherein length of formed thread at a velocity of 30 cm/min. is 20 mm or more to 200 mm or less, wherein length of formed thread at a velocity of 30 cm/min. means the length measured as follows: the surface of a sample is flatted and adjusted to be 30°C; the surface of the sample is lightly contacted with a round disk having a diameter of about 1 cm uniformly; the sample is dropped vertically at a velocity of 30 cm/min.; and the state of forming threads is observed to measure the length of thread when the thread breaks.

4. The oil-in-water external skin preparation according to any of claims 1 to 3, wherein the component (e) is one or more selected from succinoglucan, polyacrylamide, carboxyvinyl polymer, and xanthan gum.

5. The oil-in-water external skin preparation according to any of claims 1 to 4, further comprising a component (f): one or more of hydrophilic nonionic surfactants or hydrophilic anionic surfactants.

6. A production method of an oil-in-water external skin preparation according to one of claims 1-5, comprising:
after an oil phase is gradually added to an aqueous phase and the mixture is emulsified, adding and dissolving a component (a) therein,
wherein the oil-in-water external skin preparation comprising following components (a) to (d):
(a) 0.01 to 3.0 % by mass of sodium polyacrylate;
(b) 0.01 to 1.0 % by mass of alkyl-modified carboxyvinyl polymer;
(c) 1.0 to 10.0 % by mass of polyethylene glycol having an average molecular weight of 1000 or more to 50000 or less; and
(d) 0.5 to 20.0 % by mass of oil; and
wherein the oil-in-water external skin preparation has thread-forming property.

## Patentansprüche

1. Topisches Hautpräparat vom Öl-in-Wasser-Typ, enthaltend die folgenden Bestandteile (a) bis (d):
(a) 0,01 bis 3,0 Masseprozent Natriumpolyacrylat;
(b) 0,01 bis 1,0 Masseprozent alkylmodifiziertes Carboxyvinylpolymer;
(c) 1,0 bis 10,0 Masseprozent Polyethylenglycol mit einem mittleren Molekulargewicht von 1000 oder mehr bis 50000 oder weniger; und
(d) 0,5 bis 20,0 Masseprozent Öl; und wobei das topische Hautpräparat vom Öl-in-Wasser-Typ fadenziehende Eigenschaft aufweist.

2. Topisches Hautpräparat vom ÖI-in-Wasser-Typ nach Anspruch 1, ferner enthaltend einen Bestandteil (e): wasserlösliches Polymer, ausgenommen Polyacrylsäure oder eines ihrer Metallsalze und ausgenommen Bestandteil (b).

3. Topisches Hautpräparat vom Öl-in-Wasser-Typ nach Anspruch 1 oder 2, wobei die Länge eines mit einer Geschwindigkeit von 30 cm/min gezogenen Fadens 20 mm oder mehr bis 200 mm oder weniger beträgt, wobei Länge eines mit einer Geschwindigkeit von 30 cm/min gezogenen Fadens die wie folgt gemessene Länge bedeutet: Die Oberfläche einer Probe wird geglättet und auf 30 °C temperiert, die Oberfläche der Probe wird leicht mit einer runden Scheibe mit einem einheitlichen Durchmesser von 1 cm berührt, die Probe wird mit einer Geschwindigkeit von 30 cm/min senkrecht abwärts bewegt und der Vorgang des Fadenziehens wird beobachtet um die Länge des Fadens bei dessen Abreißen zu messen.

4. Topisches Hautpräparat vom Öl-in-Wasser-Typ nach einem der Ansprüche 1 bis 3, wobei der Bestandteil (e) einer oder mehrere ausgewählt aus Succinoglucan, Polyacrylamid, Carboxyvinylpolymer und Xanthangummi ist.

5. Topisches Hautpräparat vom Öl-in-Wasser-Typ nach einem der Ansprüche 1 bis 4, ferner einen Bestandteil (f): eines oder mehrere aus hydrophilen nichtionischen Tensiden oder hydrophilen anionischen Tensiden enthaltend.

6. Herstellungsverfahren für ein topisches Hautpräparat vom Öl-in-Wasser-Typ nach einem der Ansprüche 1-5, umfassend nach schrittweisem Zugeben einer Ölphase zu einer wässrigen Phase und Emulgieren der Mischung Zufügen und Auflösen eines Bestandteils (a) darin, wobei das topische Hautpräparat vom Öl-in-Wasser-Typ folgende Bestandteile enthält:
(a) 0,01 bis 3,0 Masseprozent Natriumpolyacrylat;
(b) 0,01 bis 1,0 Masseprozent alkylmodifiziertes Carboxyvinylpolymer;
(c) 1,0 bis 10,0 Masseprozent Polyethylenglycol mit einem mittleren Molekulargewicht von 1000 oder mehr bis 50000 oder weniger; und
(d) 0,5 bis 20,0 Masseprozent Öl; und wobei das topische Hautpräparat vom Öl-in-Wasser-Typ fadenziehende Eigenschaft aufweist.

## Revendications

1. Préparation topique pour la peau du type huile dans l'eau, comprenant les composants (a) à (d) suivants:
(a) 0,01 à 3,0% en masse de polyacrylate de sodium;
(b) 0,01 à 1,0% en masse d'un polymère de carboxyvinyle modifié par un alkyle;
(c) 1,0 à 10,0% en masse de polyéthylène glycol ayant un poids moléculaire moyen compris entre 1000 ou plus et 50000 ou moins; et
(d) 0,5 à 20% en masse d'huile; et
ladite préparation topique pour la peau du type huile dans l'eau présentant une propriété de formation de fils.

2. Préparation topique pour la peau du type huile dans l'eau selon la revendication 1, en outre comprenant un composant (e): un polymère hydrosoluble autre qu'un acide polyacrylique ou un sel métallique de celui-ci et autre que le composant (b).

3. Préparation topique pour la peau du type huile dans l'eau selon la revendication 1 ou 2, dans laquelle la longueur du fil formé à une vélocité de 30 cm/min est compris entre 20 mm ou plus et 200 mm ou moins, la longueur du fil formé à une vélocité de 30 cm/min désignant la longueur mesurée de la manière suivante: la surface d'un échantillon est aplatie et ajustée pour être 30°C; la surface de l'échantillon est légèrement mise en contact avec un disque rond ayant un diamètre d'environ 1 cm uniformément; on laisse tomber l'échantillon verticalement à une vélocité de 30 cm/min; et on observe l'état de formation de fils pour mesurer la longueur du fil lorsque le fil casse.

4. Préparation topique pour la peau du type huile dans l'eau selon l'une des revendications 1 à 3, dans laquelle le composant (c) est un ou plusieurs choisis parmi le succinoglycane, le polyacrylamide, le carboxyvinyle, un polymère et la gomme xanthane.

5. Préparation topique pour la peau du type huile dans l'eau selon l'une des revendications 1 à 4, en outre comprenant un composant (f): un ou plusieurs de tensioactifs non ioniques hydrophiles ou de tensioactifs anioniques hydrophiles.

6. Procédé de production d'une préparation topique pour la peau du type huile dans l'eau selon l'une des revendications 1 à 5, comprenant:
après on a ajouté graduellement à une phase aqueuse une phase huileuse et après le mélange est émulsifié, l'ajout et la dissolution d'un composant (a) dans celui-ci,
la préparation topique pour la peau du type huile dans l'eau comprenant les composants (a) à (d) suivants:
(a) 0,01 à 3,0% en masse de polyacrylate de sodium;
(b) 0,01 à 1,0% en masse d'un polymère de carboxyvinyle modifié par un alkyle;
(c) 1,0 à 10,0% en masse de polyéthylène glycol ayant un poids moléculaire moyen compris entre 1000 ou plus et 50000 ou moins; et
(d) 0,5 à 20% en masse d'huile; et
ladite préparation topique pour la peau du type huile dans l'eau présentant une propriété de formation de fils.
